# EUROPÄISCHE PATENTANMELDUNG

(11) **EP 3 404 565 A1**
(43) Veröffentlichungstag der Anmeldung: **21.11.2018**
(21) Anmeldenummer: 17171122.9
(22) Anmeldetag: 15.05.2017
(51) Int. Cl.: G06F 19/00, B60R 21/00

(54) **VERFAHREN UND SYSTEM ZUR ERZEUGUNG VON ABSCHÄTZUNGSDATEN VON POTENTIELLEN VERLETZUNGSZUSTÄNDEN**

(71) Anmelder: Siemens Healthcare GmbH, 91052 Erlangen (DE)
(72) Erfinder: Shah, Amitkumar Bhupendrakumar, 91052 Erlangen (DE); Hölzer, Philipp, 91088 Bubenreuth (DE); Ionasec, Razvan, 90402 Nuernberg (DE); Schmidt, Sebastian, 91085 Weisendorf (DE)

(57) **Zusammenfassung**

Die Erfindung betrifft ein Verfahren zur automatischen Erzeugung von Abschätzungsdaten (VD) von potentiellen Verletzungszuständen mindestens einer Person (9a, 9b) nach einem physischen Gewaltereignis, umfassend die Schritte:
- Bereitstellen von personenbezogenen Daten (PD, PD1, PD2, PD3, PD4) betreffend die Person (9a, 9b),
- Bereitstellen von Ereignisdaten (ED) betreffend das physische Gewaltereignis,
- Ermittlung der Abschätzungsdaten (VD) von zu erwartenden Verletzungszuständen mittels einer Modellierung (M) basierend auf den personenbezogenen Daten (PD, PD1, PD2, PD3, PD4) und den Ereignisdaten (ED).

Die Erfindung betrifft des Weiteren ein diesbezügliches System (1) und dazu kompatible Fahrzeuge (8c).

## Beschreibung

Die Erfindung betrifft ein Verfahren sowie ein System zur automatischen Erzeugung von Abschätzungsdaten von potentiellen Verletzungszuständen mindestens einer Person nach einem physischen Gewaltereignis

Nach einem Unfall, einer Gewalttat oder einer sonstigen physischen Gewalteinwirkung auf eine Person oder eine Personengruppe ist es wichtig, dass verletzten Personen möglichst schnell die Hilfe zuteil wird, welche die jeweiligen Verletzungen erforderlich macht.

Normalerweise werden dazu Hilfsdienste durch Notruf von einer beteiligten Person oder einem Passanten benachrichtigt.

Nachteil dieser Verfahrensweise ist, dass zwischen dem Gewaltereignis und dem Notruf unnötig wertvolle Zeit verstreicht. Zudem kann der Fall auftreten, dass ein Unfall oder eine Gewalttat ohne Zeugen an einem abgelegenen Ort erfolgt und die involvierten Personen keinen Notruf absetzen können. So kommt es zuweilen vor, dass Unfallopfer, die von einer Straße abgekommen sind, erst Stunden oder sogar erst Tage nach dem Unfall entdeckt werden.

Zukünftig ist von der Europäischen Union daher geplant, ein System namens "eCall" einzurichten, welches einen Fahrzeugunfall automatisch meldet.

Dieses System hat jedoch den Nachteil, dass zwar ein Unfall gemeldet wird, jedoch keine weiteren wichtigen Daten über die möglichen Verletzungen oder die Anzahl der involvierten Personen gesendet werden. Dadurch kann wiederum unnötig wertvolle Zeit verstreichen, bis die notwendigen Kapazitäten zum Abtransport aller Verletzten, zur Diagnose oder zur Behandlung vor Ort bzw. ausgewählt sind.

Es ist eine Aufgabe der vorliegenden Erfindung, ein verbessertes Verfahren und ein entsprechend verbessertes System zur Verfügung zu stellen, mit dem die oben beschriebenen Nachteile vermieden werden.

Diese Aufgabe wird durch ein Verfahren gemäß Patentanspruch 1 sowie durch ein System gemäß Patentanspruch 9 gelöst. Ein Fahrzeug nach Patentanspruch 13 wirkt vorteilhaft mit einem solchen System bzw. einem solchen Verfahren zusammen.

Das erfindungsgemäße Verfahren hat das Ziel eine automatische Erzeugung von Abschätzungsdaten von potentiellen Verletzungszuständen mindestens einer Person nach einem physischen Gewaltereignis zur Verfügung zu stellen.

Diese Abschätzungsdaten von potentiellen Verletzungszuständen, die im Folgenden der besseren Lesbarkeit halber auch als "Verletzungsdaten" bezeichnet werden, sind dabei Ab- oder Einschätzungen dafür, welche Verletzungen aufgetreten sein könnten. Bevorzugt umfassen sie Wahrscheinlichkeitswerte bzw. "Wahrscheinlichkeitsdaten" dafür, dass bei einer Person ein dem Verfahren bekannter Verletzungszustand vorliegt. Die Abschätzungsdaten, insbesondere Wahrscheinlichkeitswerte, können auch für zwei oder mehrere unterschiedliche mögliche Verletzungen gebildet werden. Beispielsweise können die Verletzungsdaten die Informationen umfassen, dass eine 30%ige Wahrscheinlichkeit für einen Beinbruch, eine 50%ige Wahrscheinlichkeit einer Knieverletzung und eine 90%ige Wahrscheinlichkeit einer Knöchelverstauchung vorliegt. Die Verletzungsdaten liefern somit eine Einschätzung des Gesundheitszustandes mindestens einer Person. Es ist jedoch auch möglich, dass die Abschätzungsdaten bzw. Verletzungsdaten keine quantitativen Angaben zu Wahrscheinlichkeiten betreffen, sondern qualitativ auf potentielle Verletzungen hinweisen. Beispielsweise können die Abschätzungsdaten den qualitativen Hinweis enthalten "Verdacht auf Schädelbasisbruch" o.ä.

Das physische Gewaltereignis, oder auch "physikalische" Gewaltereignis, da physikalische Kräfte wirken, ist ein Ereignis, bei dem potentiell eine Person durch Gewalt potentiell zu Schaden gekommen sein kann. Dies kann beispielsweise ein Unfall oder eine Gewalttat sein.

Das erfindungsgemäße Verfahren umfasst die Schritte:
a) Bereitstellen von personenbezogenen Daten betreffend die Person. Diese personenbezogenen Daten (oder auch "Personendaten") sind Daten, die Aufschluss über den physischen und/oder ggf. auch den psychischen Zustand bzw. die Verfassung der bei dem Gewaltereignis involvierte(n) Person(en) vor bzw. ohne Eintritt des Gewaltereignisses liefern.
b) Bereitstellen von Ereignisdaten betreffend das physische Gewaltereignis. Diese Ereignisdaten umfassen bevorzugt Messwerte und sind Daten zu verletzungsrelevanten physikalischen Einwirkungen auf die bei dem Gewaltereignis involvierte(n) Person(en).
c) Ermittlung der Abschätzungsdaten von zu erwartenden Verletzungszuständen mittels einer Modellierung basierend auf den personenbezogenen Daten und den Ereignisdaten. Diese Verletzungsdaten umfassen bevorzugt auch Informationen zu der wahrscheinlichen Stärke der Verletzungszustände. Die Modellierung umfasst bevorzugt eine Simulation oder eine Routine zur Nutzung von direkten funktionellen Zusammenhängen, insbesondere basierend auf Tabellen. Die Ergebnisse sind bevorzugt Wahrscheinlichkeitswerte für das Vorliegen von möglichen Verletzungen und bevorzugt auch deren Stärke.

Die Modellierung kann dabei eine Modellierung des gesamten Gewaltereignisses bzw. des Ereignisablaufs oder nur eines Teils des Gewaltereignisses umfassen, welcher für die Auswirkungen des Gewaltereignisses auf die involvierten Personen relevant ist, bzw. eine Modellierung der die Auswirkungen des Gewaltereignisses auf die involvierten Personen.

Eine Modellierung kann dabei z.B. die Ereignisdaten zur Abschätzung der Interaktionen möglichst vieler Objekte und Personen während des Gewaltereignisses verwenden, z.B. der Kräfte, die bei einem Unfall auf die Insassen eines Fahrzeugs, bevorzugt deren einzelne Körperteile und Organe, gewirkt haben. Die personenbezogenen Daten werden bevorzugt dazu verwendet, um abzuschätzen, welche Auswirkungen diese besagten Interaktionen auf die Physis der jeweiligen Personen gehabt haben könnten. So ist bei älteren Personen die Wahrscheinlichkeit von Knochenbrüchen in der Regel größer als bei jüngeren Personen.

Das erfindungsgemäße System dient zur automatischen Erzeugung von Abschätzungsdaten von potentiellen Verletzungszuständen mindestens einer Person nach einem physischen Gewaltereignis. Es nutzt insbesondere das oben beschriebene erfindungsgemäße Verfahren.

Das System umfasst zumindest:
a) Eine Schnittstelle zum Empfang von personenbezogenen Daten,
b) Eine Schnittstelle zum Empfang von Ereignisdaten betreffend das physische Gewaltereignis, und
c) Eine Modellierungseinheit, welche dazu ausgelegt ist, Abschätzungsdaten von zu erwartenden Verletzungszuständen mögliche Verletzungen mittels einer Modellierung basierend auf den personenbezogenen Daten und den Ereignisdaten zu ermitteln.

Ein erfindungsgemäßes Fahrzeug ist mit einem Sensorsystem zur Aufzeichnung von Ereignisdaten ausgestattet. Es ist dadurch gekennzeichnet, dass es mit einer Schnittstelle ausgestattet ist, die zur Kommunikation mit einer Schnittstelle zum Empfang von Ereignisdaten eines erfindungsgemäßen Systems ausgelegt. Zusätzlich ist das Fahrzeug bevorzugt auch mit einer Datenbank mit personenbezogenen Daten der normalerweise dieses Fahrzeug benutzenden Personen ausgestattet und umfasst zusätzlich eine Schnittstelle, die zur Kommunikation mit einer Schnittstelle zum Empfang von Ereignisdaten eines erfindungsgemäßen Systems ausgelegt ist. Dies hat den Vorteil, dass relevante Ereignisdaten und ggf. auch personenbezogene Daten sehr schnell ohne Umwege an das erfindungsgemäße System gesendet werden können bzw. dem erfindungsgemäßen Verfahren zur Verfügung stehen.

Die Abschätzungsdaten von zu erwartenden Verletzungszuständen können automatisch über bekannte Kanäle, bevorzugt über Datenleitungen oder per Funkübertragung, z.B. über das Mobilfunknetz, den betreffenden Notdiensten bereitgestellt werden. Dabei kann sich das System zusammen mit der Übertragungseinheit beispielsweise in einem beteiligten Objekt befinden, z.B. einem Fahrzeug oder als Anwendung in einem Mobilfunkgerät, aber auch an einem anderen Ort, z.B. einer zentralen Stelle, was im Grunde unerheblich ist, sofern es die notwendigen Daten erhält. Die Übertragungseinheit kann Teil des Systems sein, kann aber auch eine unabhängige Übertragungseinheit sein.

Ein Großteil der zuvor genannten Komponenten des Systems können ganz oder teilweise in Form von Softwaremodulen in einem Prozessor einer entsprechenden Steuereinrichtung realisiert werden. Eine weitgehend softwaremäßige Realisierung hat den Vorteil, dass auch schon bisher verwendete Infrastruktur auf einfache Weise durch ein Software-Update nachgerüstet werden kann, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung eines Computertomographiesystems ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Bevorzugt ist auch ein Computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Rechnereinheit ausgeführt werden.

Weitere, besonders vorteilhafte Ausgestaltungen und Weiterbildungen der Erfindung ergeben sich aus den abhängigen Ansprüchen sowie der nachfolgenden Beschreibung. Dabei können die Ansprüche einer Anspruchskategorie auch analog zu den abhängigen Ansprüchen oder Beschreibungsteilen einer anderen Anspruchskategorie weitergebildet sein und insbesondere auch einzelne Merkmale verschiedener Ausführungsbeispiele bzw. Varianten zu neuen Ausführungsbeispielen bzw. Varianten kombiniert werden.

Zudem können Fahrzeuge auch einzelne Komponenten des erfindungsgemäßen Systems oder ein vollständiges System enthalten und damit nach einem Unfall autark die vorbestimmten Informationen liefern, was insbesondere bei Unfällen an abgelegenen Orten einen großen Vorteil darstellt.

Bevorzugt enthalten die personenbezogenen Daten biometrische Daten, Daten zur Identität und/oder zur körperlichen Verfassung, insbesondere bekannte Vorerkrankungen. Bevorzugte personenbezogene Daten sind Daten der Gruppe Alter, Geschlecht, Größe, Gewicht, Vorerkrankungen und Daten einer Krankenakte. Bereits aus diesen Daten können wesentliche Aussagen über die körperliche Verfassung einer Person abgeleitet werden.

Bevorzugt enthalten die Ereignisdaten Daten über einen oder mehrere der folgenden Fälle. Dabei kann es sich beispielsweise jeweils um rein numerische Daten handeln oder Daten eines semantischen Datenmodells. Bei dem Format der Daten sollte dies so gewählt sein, dass im Modellierungsschritt diese Daten bearbeitet bzw. in einen relevanten Kontext zum Ergebnis gesetzt werden können.
i) Ereignisbeschreibung: Dies sind Daten zur Ereignisart. Beispielsweise, ob es sich um einen Autounfall, einen Motorradunfall, einen Unfall mit Fußgängern, eine Messerstecherei, einen Schusswechsel, stumpfe Gewalteinwirkung, einen Sturz oder weitere mögliche Gewaltereignisse handelt.
ii) Geschwindigkeit, Bewegungsrichtung, relative Positionen, Beschleunigungen/einwirkende Kräfte auf die Person oder sonstige physikalische Gegebenheiten bei dem Gewaltereignis. Dabei können die betreffenden Daten der involvierten Personen wichtige Hinweise zu potentiellen Verletzungen liefern, aber auch betreffende Daten von Objekten, z.B. Fahrzeugen oder Daten von Objekten, welche Personen verletzt haben könnten (z.B. von Pistolenkugeln).
iii) Relative Orientierung der Person(en) und/oder von involvierten Objekten. Diese Daten können vorteilhaft zur Einschätzung genutzt werden, welche Objekte mit welchen Personen interagiert haben könnten.
iv) Beschreibung von involvierten Objekten. Diese können wichtige Hinweise auf die Art von potentiellen Verletzungen liefern, z.B. ob ein Messer, eine Kugel oder ein stumpfer Gegenstand die Verletzung hervorgerufen haben könnte.
v) Partialbeschleunigungen der Körperteile der Person oder Eindringtiefen von Objekten in diese Person. Diese Daten sind hilfreich, um abzuschätzen, welche Regionen des Körpers welche Verletzungen aufweisen könnten.
vi) Physischer Einfluss von mehreren dem Gewaltereignis unterworfenen Personen auf mindestens eine der involvierten Personen. Diese Daten sind hilfreich, um Verletzungsmuster abzuleiten, die durch die Interaktion von Körperteilen unterschiedlicher Personen aufeinander entstanden sein könnten, z.B. Fausthiebe aber auch eine mögliche Penetration von Organen einer Person durch einen offenen Bruch einer anderen Person.

Bevorzugt umfassen Ereignisdaten insbesondere eines Fahrzeugunfalls Daten der Gruppe Fahrzeugtyp (Gewichtsklasse, Form), Geschwindigkeit, Aufprallwinkel, Orientierung eines Fahrzeugs zum Opfer, Beschaffenheit der Motorhaube bzw. der Fahrzeugkarosserie (glatt oder hervorstehende Objekte wie z.B. ein Emblem oder ein Frontschutzbügel), Deformationen des Fahrzeugs an der Aufprallstelle und die Aufprallstelle an der Person (Hüfte, Brustkorb, Bein, etc.)

Bevorzugt werden die Ereignisdaten von Sensoren oder Personen erfasst bzw. erhalten. Eine Möglichkeit ist dabei, dass die Ereignisdaten Messwerte von Sensoren umfassen, die mit zumindest einem beim physischen Gewaltereignis beteiligten Objekt gekoppelt sind. Solche Sensoren sind z.B. Sensoren in einem Fahrzeug oder einem von einer Person mitgeführten Mobilfunkgerät bzw. einer Smartwatch oder Fitnesssensoren. Die Ereignisdaten können aber auch Aufnahmedaten zumindest einer das physische Gewaltereignis aufzeichnende Aufnahmeeinheit, z.B. einer Verkehrskamera, enthalten oder Zeugenaussagen bzw. Videomitschnitte von Zeugen enthalten. Aus Videomitschnitten des Gewaltereignisses können beispielsweise der Tat- bzw. Unfallhergang ermittelt werden, sowie Geschwindigkeiten abgeschätzt werden. Ähnliches gilt für Zeugenaussagen, die mittels Spracherkennungssystemen analysiert werden können.

Bevorzugt sind auch weitere Arten von Sensoren, insbesondere der Gruppe Sensoren zur Überwachung eines Menschen, z.B. Geräte zur Messung der Sauerstoffsättigung im Blut oder zur Aufnahme eines EKG oder EEG, Sensoren der Messung von Beschleunigung oder sonstige mechanischen Größen, Sensoren zur Messung der Temperatur, Sensoren zur Messung von Geräuschen oder Stimmen, z.B. Mikrofone, oder Sensoren welche das Auslösen eines Airbags detektieren können, z.B. Sensoren in der Auslöseelektronik eines Fahrzeugairbags.

Bevorzugt werden Abschätzungsdaten von potentiellen Verletzungszuständen mehrerer Personen erzeugt, wobei insbesondere die Interaktion der Personen bei dem physischen Gewaltereignis bei der Modellierung berücksichtigt wird. Auf diese Weise können Verletzungsmuster modelliert werden, die unter anderem von anderen beteiligten Personen hervorgerufen werden. Die Modellierung kann dabei ausgehend von jeder der involvierten Person nacheinander vorgenommen werden oder ausgehend von einem Teil oder allen Personen zur gleichen Zeit. Dies könnte insbesondere durch Lösung einer zu minimierenden Kostenfunktion erfolgen.

Die Modellierung umfasst dabei bevorzugt Berechnungen, welche die Interaktion der Personen im Hinblick auf die Abschätzungsdaten von zu erwartenden Verletzungszuständen der betreffenden Personen und/oder der übrigen Personen betreffen.

Bevorzugt umfasst die Erzeugung von Abschätzungsdaten von potentiellen Verletzungszuständen mit Hilfe der Modellierung eine Simulation und/oder eine verzeichnisbasierte Suche bzw. eine Auswahl von Einträgen aus einem Verzeichnis ("Dictionary") gemäß den vorliegenden personenbezogenen Daten und Ereignisdaten umfasst. Bevorzugt sind in diesem Rahmen Kalkulationen basierend auf finiten Elementen, Differenzen oder Volumina, Montecarlo-Simulationen oder Markow-Ketten. Ebenfalls bevorzugt sind graphische und/oder volumenbasierte Simulationen mittels als Volumenkörper modellierter Personen bzw. Objekten und einer Simulation von auf Ereignisdaten basierender physikalischer Gesetzmäßigkeiten. Bevorzugt ist auch eine Modellierung basierend auf Prinzipien des Maschinellen Lernens. Insbesondere wird in dieser Hinsicht ein Algorithmus zunächst mit Datensätzen umfassend Ereignisdaten und personenbezogene Daten trainiert. Dieser trainierte Algorithmus liefert dann auf Basis der jeweils aktuellen personenbezogenen Daten und Ereignisdaten in Abhängigkeit seines Trainingsstandes Verletzungsdaten. Allgemein soll der Algorithmus inhärent den Zusammenhang zwischen Situation und Ergebnis darstellen.

Eine bevorzugte Modellierung wird im Folgenden am Beispiel eines Autounfalls genauer erklärt.
a) Zunächst erfolgt die Eingabe von Informationen über den Unfallhergang und die involvierten Personen. Dazu werden die Ereignisdaten und die personenbezogenen Daten verwendet. Solche Daten können beispielsweise Daten zum Fahrzeugtyp, zur Geschwindigkeit, dem Aufprallwinkel, der Orientierung zum Opfer, Deformationen des Fahrzeugs und die Aufprallstelle an der Person sein.
b) Dann erfolgt die Bereitstellung einer Datenbank mit Daten zu Simulationen, welche aus verfügbaren Informationen zu anderen Gewaltereignissen vorher durchgeführt worden sind. Diese Simulationen umfassen dabei sowohl den Unfallhergang bzw. die Szenerie des Gewaltereignisses als auch die medizinische Auswirkung auf die bei den betreffenden anderen Gewaltereignissen involvierten Personen.
c) Es erfolgt ein Abgleich der Informationen (Ereignisdaten und personenbezogene Daten) zu dem aktuellen Gewaltereignis mit Einträgen aus der vorgenannten Datenbank, um eine ähnliche Unfallsituation zu ermitteln und entsprechende Simulationen wie bei dem ähnlichen Unfall durchzuführen bzw. die entsprechenden Daten aus der Datenbank zu erhalten.
d) Damit ist eine Vorhersage der medizinischen Auswirkungen auf Unfallopfer basierend auf den Daten der Datenbank bzw. der von der Datenbank vorgegebenen Simulation möglich.

Eine bevorzugte Datenbank mit Simulationen einer Kollision mit einem Objekt bzw. Gegenstand umfasst simulierte Daten einer Kollision aus vielen verschiedenen (ggf. allen) möglichen Raumrichtungen, eine Kollision mit vielen verschiedenen (ggf. allen) möglichen Formen und eine Kollision mit vielen verschiedenen (ggf. allen) möglichen Materialien.

Bevorzugt basieren Simulationen auf Informationen der Gruppe umfassend
- typische mechanische Eigenschaften von Organen, wie z.B. Deformationen, Knochenbrüche, Gefäßrisse,
- mechanische Eigenschaften von Werkstoffen, wie z.B. Splittern, Kantenbildung, Festigkeit, die in oder an involvierten Objekten, z.B. Automobilen, verwendet werden,
- Position von Organen im Körper einer Person,
- chemische Verträglichkeit menschlichen Geweben mit am Gewaltereignis beteiligten Werkstoffen, und
- Biomechanik des Körpers, wie z.B. der Wirbelsäule zur Abschätzung, ob nach einem Gewaltereignis möglicherweise ein Schleudertrauma vorliegen könnte.

Geeignete Randbedingungen basieren auf der Energieerhaltung und der Impulserhaltung, sowie elastischer Eigenschaften von Materialien, welche der Dämpfung von Beschleunigungen dienen können und Reibungskoeffizienten.

Bevorzugt werden basierend auf den ermittelten Abschätzungsdaten von potentiellen Verletzungszuständen, und ggf. deren wahrscheinlicher Stärke, geeignete Untersuchungsverfahren und/oder Behandlungsverfahren zur gezielten Untersuchung und/oder Behandlung der Person ermittelt. Bevorzugt ist in diesem Rahmen eine Ermittlung der Gruppe Priorisierung der voraussichtlich notwendigen Untersuchungen, Abschätzung der Schwere der Verletzungen und Empfehlung für erforderliche Interventionen.

Bevorzugt wird aus den Verletzungsdaten ein Protokoll für eine Steuerung eines medizintechnischen Geräts erstellt, z.B. zur Steuerung eines Computertomographen ("CT"), eines Magnetresonanztomographen ("MRT"), eines Positron-Elektron-Tomographen ("PET"), eines Geräts zur Einzelphotonen-Emissionscomputertomographie ("SPECT"), eines Geräts zur Durchleuchtung eines Körpers, eines Gerätes für Ultraschallaufnahmen, eines Geräts zur optischen Kohärenztomografie ("OCT"), eines Geräts zur Photoakustik oder eines Geräts zur Ultra-Breitband Bildgebung ("UWB Imaging"). Dies beschleunigt eine spätere Diagnose durch einen Arzt, da die Steuerdaten für das Gerät nicht erst erstellt werden müssen. Ein bereits erstellter Steuerdatensatz kann auch durch einen Arzt basierend auf sein Fachwissen einfach und zeitschonend an neue Erkenntnisse angepasst werden.

Das System umfasst hierzu bevorzugt eine Ermittlungseinheit die basierend auf den ermittelten möglichen Verletzungen, und ggf. deren wahrscheinlicher Stärke, sowie bevorzugt basierend auf ermittelten geeigneten Untersuchungsverfahren, Kapazitäten von Rettungs- und Diagnoseeinrichtungen automatisch ermittelt.

Bevorzugt werden basierend auf den ermittelten Abschätzungsdaten von potentiellen Verletzungszuständen, und ggf. deren wahrscheinlicher Stärke, sowie bevorzugt basierend auf ermittelten geeigneten Untersuchungsverfahren, wahrscheinlich erforderliche Kapazitäten von Rettungs- und/oder Untersuchungseinrichtungen ermittelt und vorzugsweise reserviert und insbesondere auch benachrichtigt. Dies hat den Vorteil, dass eine schnelle Verteilung von Opfern auf freie Rettungs- und Untersuchungskapazitäten und dadurch eine schnelle Behandlung erfolgen kann.

Das System umfasst hierzu bevorzugt eine Benachrichtigungseinheit, die basierend auf den ermittelten möglichen Verletzungen, und ggf. deren wahrscheinlicher Stärke, sowie insbesondere basierend auf ermittelten geeigneten Untersuchungsverfahren, Kapazitäten von Rettungs- und Diagnoseeinrichtungen automatisch reserviert und/oder benachrichtigt. Diese Benachrichtigung kann beispielsweise über die nachfolgend erwähnten bekannten Kanäle erfolgen. Insbesondere kann ein eCall-System mit der vorliegenden Erfindung verbessert werden und dessen Übertragungskanäle verwendet werden.

Im Rahmen der Benachrichtigung können die ermittelten Abschätzungsdaten potentieller Verletzungszustände bevorzugt zusammen mit Informationen über die involvierten Objekte oder Personen, insbesondere die erwarteten Opferzahlen, übermittelt werden.

Bevorzugt ist die Schnittstelle zum Empfang von Ereignisdaten des Systems zum Zugriff auf ein Rechensystem ausgelegt, bevorzugt über das Internet of Things (IoT), eine Computercloud, Drahtlosnetzwerke, z.B. 4G, 5G, oder Internetprotokolle, z.B. IPv6. Dies ermöglicht einen schnellen und unkomplizierten Erhalt der benötigten Ereignisdaten.

Bevorzugt ist die Schnittstelle zum Empfang von personenbezogenen Daten zum Zugriff auf Speichermedien, bevorzugt Datenbanken, umfassend elektronische Patientendaten, vorzugsweise gemäß HL7-Standards, oder auf Systeme zum medizinischen Bildtransfer, bevorzugt gemäß DICOM-Standards (DICOM = Digital Imaging and Communications in Medicine = Digitale Bildverarbeitung und Kommunikation in der Medizin), ausgelegt.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Darstellung einer bevorzugten Ausführungsform eines erfindungsgemäßen Verfahrens,
Figur 2 eine schematische Darstellung einer bevorzugten Ausführungsform eines erfindungsgemäßen Systems,
Figur 3 ein Schema zur Verdeutlichung von möglichen Arten von personenbezogenen Daten,
Figur 4 ein Schema zur Verdeutlichung von möglichen Arten von Ereignisdaten und deren Bereitstellung,

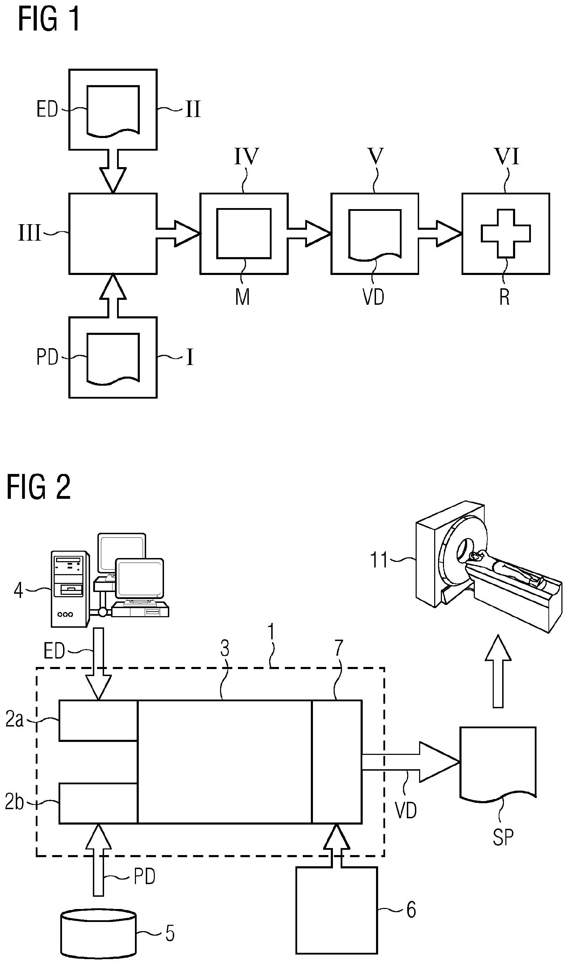

Figur 1 verdeutlicht ein bevorzugtes Verfahren mittels eines Blockschaltbildes.

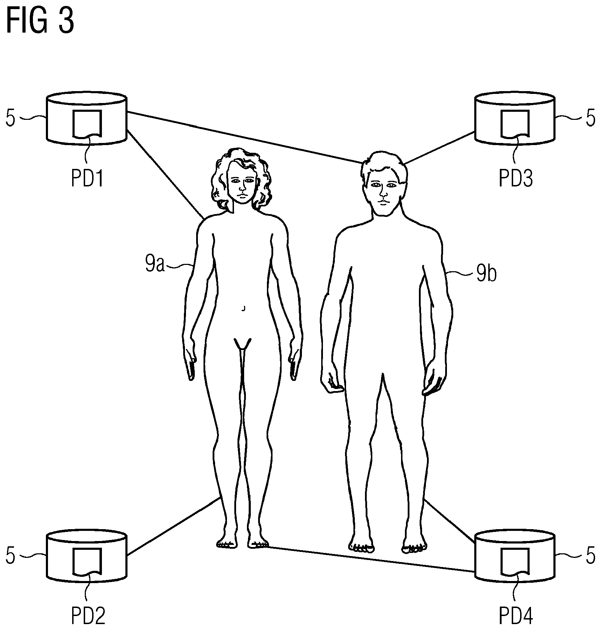

Block I zeigt die Bereitstellung personenbezogener Daten PD für das Verfahren. Die personenbezogenen Daten PD können wie in Figur 3 angedeutet wird, unterschiedliche personenbezogene Daten PD1, PD2, PD3 oder PD4 sein.

In Figur 3 wird die Aufteilung unterschiedlicher personenbezogener Daten PD1, PD2, PD3 und PD4 von zwei Personen 9a und 9b auf verschiedenen Datenbanken 5 skizziert. Wie in den Datenbanken 5 links oben und rechts unten zu sehen, können personenbezogene Daten PD1, PD4 beider Personen in denselben Datenbanken vorliegen, z.B. wenn die Personen miteinander familiär verbunden sind und/oder bei demselben Arzt in Behandlung sind, sie können aber auch unabhängig voneinander in unterschiedlichen Datenbanken 5 vorliegen. Beispielsweise sind die Daten PD1 Daten zur Identität der Personen, z.B. Name, Adresse, Alter. Diese können durchaus bei familiär verbundenen Menschen in einer Datei einer Datenbank vorliegen. Genauso können die personenbezogenen Daten PD4 für beide Personen in einer gemeinsamen Datenbank vorliegen, die beispielsweise biometrische Daten wie z.B. Größe oder Gewicht umfassen. Die Daten PD2 und PD3 sind beispielsweise medizinische Daten der jeweiligen Personen, die in getrennten Datenbanken vorliegen, weil diese Personen in diesem Beispiel bei unterschiedlichen Ärzten in Behandlung sind.

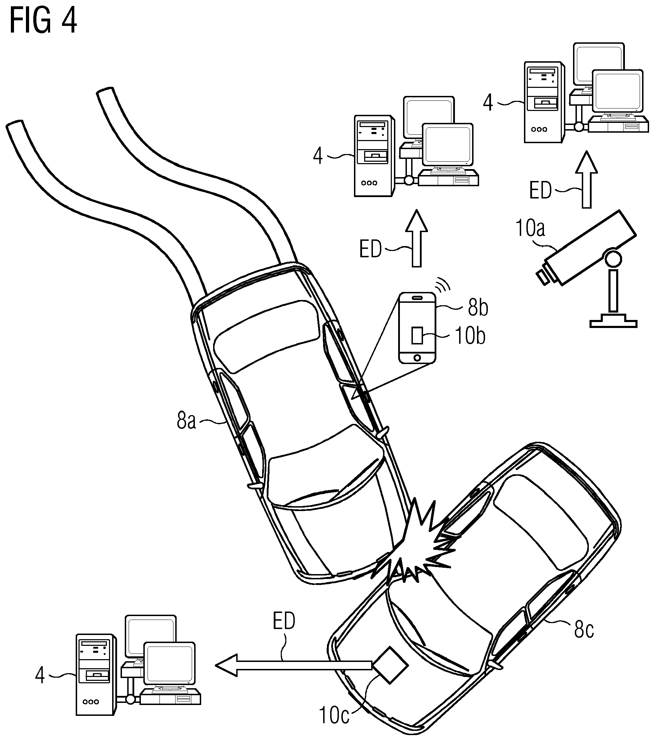

Block II zeigt die Bereitstellung von Ereignisdaten ED für das Verfahren. Die Ereignisdaten ED können, wie in der noch später genauer erklärten Figur 4 angedeutet wird, von unterschiedlichen Sensoren 10a, 10b, 10c erhalten werden. In der Regel nimmt eine Instanz die Daten auf. Diese Instanzen sind z.B. Mobilfunkunternehmen, Verkehrsüberwachung, Fahrzeugmanagement. Die Daten werden zumeist in Rechensystemen 4 abgespeichert, welche, ggf. auf Anfrage, diese Daten bereitstellen. Somit können die unterschiedlichen Ereignisdaten ED, auf die das Verfahren zugreift, von mehreren unterschiedlichen Rechensystemen 4 stammen bzw. von diesen bereitgestellt werden.

Block III verdeutlicht die Aufbereitung der Daten in ein Format, welches eine Modellierung M erlaubt. Dieser Schritt ist nicht unbedingt notwendig, zumindest wenn die Daten ED bzw. PD bereits in einem geeigneten Format vorliegen oder die Modellierung zur Bearbeitung von den jeweiligen Formaten ausgelegt ist.

Block IV symbolisiert den Modellierungsschritt. Hier findet die Modellierung M basierend auf den personenbezogenen Daten PD und den Ereignisdaten ED statt, z.B. eine Simulation des Unfallhergangs. Es ergeben sich unter Nutzung der Modellierung die Verletzungsdaten VD.

Diese Modellierung verwendet die Ereignisdaten zur Abschätzung der Interaktionen von Personen und Objekten während des Gewaltereignisses. Beispielsweise werden aus Geschwindigkeiten bzw. Beschleunigungen Kräfte extrapoliert, die bei einem Unfall auf die Insassen eines Fahrzeugs, ggf. partiell auf Körperregionen, gewirkt haben. Zur Abschätzung der daraus potentiell resultierenden Verletzungen werden die personenbezogenen Daten verwendet, die Aufschluss darüber geben können, welche Wirkung die auf eine Person wirkenden Kräften auf deren Körper haben könnten.

Im Block V werden die Verletzungsdaten VD bereitgestellt. Dies kann z.B. zur Ermittlung von Untersuchungsverfahren und/oder Behandlungsverfahren zur gezielten Untersuchung und/oder Behandlung der Person (9a, 9b) oder zur Erstellung eines Protokolls SP für eine Steuerung eines medizintechnischen Geräts, wie z.B. eines Tomographen 11 verwendet werden.

In Block VI wird die Ermittlung und Reservierung von wahrscheinlich erforderlicher Kapazitäten von Rettungseinrichtungen R und/oder Untersuchungseinrichtungen basierend auf den Verletzungsdaten VD dargestellt. Beispielsweise werden Rettungsdienste entsprechend der Anzahl der ermittelten potentiellen Opfer und der zu erwartenden Verletzungen informiert und ggf. bereits Betten in Krankenhäusern reserviert. Es könnte jedoch z.B. auch ein Steuerungsprotokoll für einen Computertomographen versendet und werden, welches eine schnelle Untersuchung einer verletzten Person gleich nach ihrer Einlieferung in die entsprechende Einrichtung erlaubt.

Figur 2 stellt ein bevorzugtes System 1 schematisch dar. Die personenbezogenen Daten PD sind hier in einer Datenbank 5 abgespeichert, die wie bereits oben bezüglich der Figur 3 erwähnt wurde, auch eine Gruppe von unterschiedlichen Datenbanken 5 symbolisieren kann. Die Ereignisdaten ED sind in einem Rechensystem 4 hinterlegt, das wie bereits oben bezüglich der Figur 4 erwähnt wurde, auch eine Gruppe von unterschiedlichen Rechensystemen 4 symbolisieren kann.

Es erfolgt eine Bereitstellung der personenbezogenen Daten PD durch die Datenbank 5 über die Schnittstelle 4b und eine Bereitstellung der Ereignisdaten ED mittels des Rechensystems 4 über die Schnittstelle 4a an das System 1. Diese Daten können ggf. umformatiert oder aufbereitet werden, was bezüglich des Verfahrens bereits erwähnt wurde, jedoch hier nicht extra eingezeichnet ist.

In der Modellierungseinheit 3 werden die Verletzungsdaten VD durch eine Modellierung M der Auswirkungen des Gewaltereignisses auf die involvierten Personen erzeugt. Diese Verletzungsdaten VD können dann mittels einer Benachrichtigungseinheit 7 Diensten zur Verfügung gestellt werden, wie es im Zuge des Verfahrens bereits beschrieben wurde. Dies kann basierend auf durch eine Ermittlungseinheit 6 ermittelten Daten geschehen. Diese Ermittlungseinheit 6 kann beispielsweise dem System mitteilen, in welchem Krankenhaus welche Kapazitäten vorliegen bzw. frei sind.

Dargestellt ist hier die Bereitstellung von Verletzungsdaten VD zur Erzeugung eines Steuerprotokolls SP für einen Tomographen 11. Das Steuerprotokoll SP kann dabei extern erzeugt werden, es kann aber auch mittels des Systems 1 direkt erzeugt werden.

In Figur 4 ist ein Gewaltereignis in Form eines Autounfalls dargestellt. Ein Unfallfahrzeug 8a ist in die Seite eines anderen Unfallfahrzeugs 8c gefahren. In dem Fahrzeug 8a befindet sich ein Mobilfunkgerät 8b, welches beispielsweise vom Fahrer getragen worden sein könnte und ggf. auch Verletzungen hervorgerufen haben könnte. Die Unfallfahrzeuge und das Mobilfunkgerät können auch als "Objekte" im Sinne der Erfindung bezeichnet werden.

In dem Mobilfunkgerät 8b befindet sich ein Beschleunigungssensor 10b, der Daten an ein Rechensystem 4 des Mobilfunkbetreibers gesendet hat. Darunter sind auch Ereignisdaten ED, die während des Unfalls aufgezeichnet worden sind, und Aufschluss über die Beschleunigung des Fahrers während des Unfalls geben könnten.

In dem Fahrzeug 8c befindet sich zudem ein Fahrzeugsensor 10c, der den jeweiligen Zustand des Fahrzeugs 8c, u.a. auch auf dieses Fahrzeug 8c wirkende Beschleunigungen misst. Die Daten sendet der Fahrzeugsensor 10c an ein Rechensystem 4 des Fahrzeugherstellers. Darunter sind ebenfalls Ereignisdaten ED, die während des Unfalls aufgezeichnet worden sind, und Aufschluss über die Geschwindigkeit und Beschleunigung des Unfallfahrzeugs 8c während des Unfalls geben könnten.

Als drittes wurde dieser Unfall von einer Verkehrskamera 10a aufgezeichnet, deren Daten, unter anderem auch die Ereignisdaten ED, an ein Rechensystem 4 der Verkehrsüberwachung gesendet wurden.

All die Ereignisdaten ED der unterschiedlichen Rechensysteme 4 können von dem erfindungsgemäßen Verfahren bzw. dem erfindungsgemäßen System zur Modellierung M der Auswirkungen des Gewaltereignisses auf die involvierten Personen verwendet werden. Je mehr Daten dem erfindungsgemäßen System 1 bzw. Verfahren zur Verfügung stehen, desto akkurater wird das Ergebnis sein.

Es wird abschließend noch einmal darauf hingewiesen, dass es sich bei den vorhergehend detailliert beschriebenen Verfahren sowie bei dem dargestellten System 1 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriff "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur automatischen Erzeugung von Abschätzungsdaten (VD) von potentiellen Verletzungszuständen mindestens einer Person (9a, 9b) nach einem physischen Gewaltereignis, umfassend die Schritte:
- Bereitstellen von personenbezogenen Daten (PD, PD1, PD2, PD3, PD4) betreffend die Person (9a, 9b),
- Bereitstellen von Ereignisdaten (ED) betreffend das physische Gewaltereignis,
- Ermittlung der Abschätzungsdaten (VD) von zu erwartenden Verletzungszuständen mittels einer Modellierung (M) basierend auf den personenbezogenen Daten (PD, PD1, PD2, PD3, PD4) und den Ereignisdaten (ED).

2. Verfahren nach Anspruch 1, wobei die personenbezogenen Daten (PD, PD1, PD2, PD3, PD4) Daten der Gruppe Alter, Geschlecht, Größe, Gewicht, Vorerkrankungen und Daten einer Krankenakte enthalten.

3. Verfahren nach einem der vorangehenden Ansprüche, wobei die Ereignisdaten (ED) Daten der Gruppe
- Ereignisbeschreibung
- Geschwindigkeit,
- Bewegungsrichtung,
- relative Positionen,
- relative Orientierung einer Person oder von Personen (9a, 9b) und/oder involvierten Objekten (8a, 8b, 8c),
- Beschreibung von involvierten Objekten (8a, 8b, 8c),
- einwirkende Kräfte und/oder Beschleunigungen auf Personen (9a, 9b),
- Partialbeschleunigungen von Körperteilen von Personen (9a, 9b),
- Eindringtiefe von Objekten in Personen (9a, 9b), und
- physischer Einfluss von mehreren dem Gewaltereignis unterworfenen Personen (9a, 9b) auf mindestens eine der Personen (9a, 9b)
enthalten.

4. Verfahren nach einem der vorangehenden Ansprüche, wobei die Ereignisdaten (ED)
- mittels Messwerten von Sensoren (10b, 10c), die vorzugsweise mit zumindest einem beim physischen Gewaltereignis beteiligten Objekt (8a, 8b, 8c) gekoppelt sind
und/oder
- mittels Aufnahmedaten zumindest einer das physische Gewaltereignis aufzeichnenden Aufnahmeeinheit (10a)
und/oder
- mittels Zeugenaussagen zum physischen Gewaltereignis erhalten und bereitgestellt werden.

5. Verfahren nach einem der vorangehenden Ansprüche, welches Abschätzungsdaten (VD) von potentiellen Verletzungszuständen mehrerer Personen (9a, 9b) erzeugt,
wobei bevorzugt die Interaktion der Personen (9a, 9b) bei dem physischen Gewaltereignis bei der Modellierung (M) berücksichtigt wird.

6. Verfahren nach einem der vorangehenden Ansprüche, wobei die Erzeugung von Abschätzungsdaten (VD) von potentiellen Verletzungszuständen mit Hilfe der Modellierung (M) eine Simulation und/oder eine verzeichnisbasierte Suche umfasst.

7. Verfahren nach einem der vorangehenden Ansprüche, wobei basierend auf den ermittelten Abschätzungsdaten (VD) von potentiellen Verletzungszuständen geeignete Untersuchungsverfahren und/oder Behandlungsverfahren zur gezielten Untersuchung und/oder Behandlung der Person (9a, 9b) ermittelt werden,
wobei bevorzugt eine Priorisierung der voraussichtlich notwendigen Untersuchungen, eine Abschätzung der Schwere der Verletzungen und/oder eine Empfehlung für erforderliche Interventionen, vorgenommen wird,
und/oder wobei bevorzugt ein Protokoll (SP) für eine Steuerung eines medizintechnischen Geräts (11) erstellt wird.

8. Verfahren nach einem der vorangehenden Ansprüche, wobei basierend auf den ermittelten Abschätzungsdaten (VD) von potentiellen Verletzungszuständen wahrscheinlich erforderliche Kapazitäten von Rettungs- und/oder Untersuchungseinrichtungen (R) ermittelt und vorzugsweise reserviert werden.

9. System (1) zur automatischen Erzeugung von Abschätzungsdaten (VD) von potentiellen Verletzungszuständen mindestens einer Person (9a, 9b) nach einem physischen Gewaltereignis, bevorzugt mittels eines Verfahrens nach einem der vorangehenden Ansprüche, umfassend
- eine Schnittstelle (2b) zum Empfang von personenbezogenen Daten (PD, PD1, PD2, PD3, PD4),
- eine Schnittstelle (2a) zum Empfang von Ereignisdaten (ED) betreffend das physische Gewaltereignis,
- eine Modellierungseinheit (3), welche dazu ausgelegt ist, Abschätzungsdaten (VD) von zu erwartenden Verletzungszuständen mögliche Verletzungen mittels einer Modellierung (M) basierend auf den personenbezogenen Daten (PD, PD1, PD2, PD3, PD4) und den Ereignisdaten (ED) zu ermitteln.

10. System nach Anspruch 9, wobei die Schnittstelle (2a) zum Empfang von Ereignisdaten (ED) zum Zugriff auf ein Rechensystem (4), bevorzugt über das Internet of Things (IoT), eine Computercloud, Drahtlosnetzwerke oder Internetprotokolle, ausgelegt ist.

11. System nach Anspruch 9 oder 10, wobei die Schnittstelle (2b) zum Empfang von personenbezogenen Daten (PD, PD1, PD2, PD3, PD4) zum Zugriff auf Speichermedien (5) umfassend elektronische Patientendaten, bevorzugt gemäß HL7-Standards, und/oder zum Zugriff auf Systeme zum medizinischen Bildtransfer, bevorzugt gemäß DICOM-Standards, ausgelegt ist.

12. System nach einem der Ansprüche 8 bis 10 mit einer Ermittlungseinheit (6) und/oder Benachrichtigungseinheit (7), die basierend auf den ermittelten möglichen Verletzungen (VD), bevorzugt basierend auf ermittelten geeigneten Diagnoseverfahren, Kapazitäten von Rettungs- und Diagnoseeinrichtungen (R) automatisch ermittelt, reserviert und/oder benachrichtigt.

13. Fahrzeug (8c) mit einem Sensorsystem (10c) zur Aufzeichnung von Ereignisdaten (ED), welches mit einer Schnittstelle ausgestattet ist, die zur Kommunikation mit einer Schnittstelle (2a) zum Empfang von Ereignisdaten (ED) eines Systems (1) nach einem der Ansprüche 9 bis 12 ausgelegt ist, und/oder wobei das Fahrzeug dazu ausgelegt ist, Ereignisdaten ED für ein Verfahren gemäß einem der Ansprüche 1 bis 8 zur Verfügung zu stellen.

14. Computerprogrammprodukt mit einem Computerprogramm, welches direkt in eine Speichereinrichtung einer Rechnereinrichtung (8b, 4) ladbar ist, mit Programmabschnitten, um alle Schritte des Verfahrens nach einem der Ansprüche 1 bis 8 auszuführen, wenn das Computerprogramm in der Rechnereinrichtung ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 8 auszuführen, wenn die Programmabschnitte von der Rechnereinheit ausgeführt werden.
